# DEMANDE DE BREVET EUROPEEN

(11) **EP 1 980 906 A1**
(43) Date de publication de la demande: **15.10.2008**
(21) Numéro de dépôt: 08154445.4
(22) Date de dépôt: 11.04.2008
(51) Int. Cl.: G03B 21/56, G03B 29/00, A61M 21/00, E04H 3/22, A61H 33/00

(54) **Dispositif de restitution d'images**

(30) Priorité: 13.04.2007 FR 0754461
(71) Demandeur: Caradec, Stéphanie, 75005 Paris (FR)
(72) Inventeur: Caradec, Stéphanie, 75005 Paris (FR)
(74) Mandataire: Becker, Philippe

(57) **Abrégé**

L'invention concerne un dispositif de restitution d'images (1), comprenant :
- une chambre sourde ;
- un écran d'affichage (7) disposé dans la chambre sourde ;
- une assise (5) disposée dans la chambre sourde de sorte qu'un utilisateur puisse visionner l'écran; et
-de manière facultative, une tenue vestimentaire spécifique.

## Description

L'invention concerne les dispositifs de restitution d'images, et en particulier les dispositifs de restitution d'images destinés au public.

Le mode de vie urbain développe le nombre de sources de pollution sonore. Le niveau du bruit ambiant est ainsi globalement en croissance assez forte. Un tel bruit ambiant est source de stress et de fatigue.

Différents dispositifs ont été envisagés pour tenter de soulager cette fatigue. Ainsi, le document FR2,749,515 porte sur un dispositif de détente et de relaxation mettant en oeuvre un caisson d'isolation sensorielle. Le document GB2139490 décrit une chambre de flottaison dans laquelle une personne peut s'enfermer pour se détendre, et le document EP 1 260 855 concerne un écran incurvé pour la projection d'images. Le document JP11 212441 concerne une salle d'expérimentation qui permet de comparer différents "états" d'isolation comportant trois parois isolantes et un plafond semi-isolant et la présence de drains, et le brevet US4,640,266 concerne un appareil de stimulation sensorielle comprenant une pièce dans laquelle différents stimuli, par exemple optiques, sonores, olfactifs, etc., peuvent être émis.

L'invention propose, pour la première fois, un dispositif de restitution d'images mettant en oeuvre une chambre sourde. A la différence de l'ensemble des dispositifs antérieurs, le dispositif de l'invention met en oeuvre une chambre sourde, c'est-à-dire ayant des propriétés acoustiques particulières, et permet de transformer la perception visuelle, produisant sur chacun une sensation d'immersion et de réalité virtuelle. La réalité virtuelle est une simulation interactive immersive, visuelle, sonore et/ou haptique, d'environnements réels ou imaginaires. L'invention propose ainsi un dispositif de restitution d'images dans un silence profond (le degré 0 du son, s'il peut être expérimentalement produit dans un programme de déprivation sensorielle, n'existe pas dans la nature), et montre que, au delà de la sensation de détente provoquée, la perception d'images dans un silence profond aborde des notions d'esthétiques et rapproche la vision de l'utilisateur, de celle générée par le cerveau en phase d'endormissement (perceptions visuelles ne correspondant pas à la réalité) caractérisée par une sensation de flottement, une vision holographique, mouvante et onirique.

L'invention permet ainsi d'accroître l'immersion de l'utilisateur, du fait d'effets sur les hémisphères du cerveau induisant une perception visuelle totalement nouvelle.

L'invention porte ainsi sur un dispositif de restitution d'images, comprenant :
- une chambre sourde dite anéchoïque ou semi-anéchoïque;
- un écran d'affichage disposé dans la chambre sourde ; et
- une assise disposée dans la chambre sourde de sorte qu'un utilisateur puisse visionner l'écran.

Selon une variante, le dispositif comprend une tenue vestimentaire spécifique (par exemple une combinaison, blouse, ou un gilet sans manche), conçue en matériau(x) adapté(s) et absorbant(s), permettant de diminuer les sons émis par le corps de l'utilisateur (gargouillements d'estomac, battements cardiaques, etc.) dans la chambre sourde. Cette tenue permet également que tous les individus entrant dans le dispositif soient vêtus pareillement afin d'obtenir une perception la plus similaire pour chacun des spectateurs et ainsi préciser l'application.

Selon une variante, le dispositif comprend une plate-forme sur laquelle est fixée l'assise, la plate-forme étant suspendue au dessus d'un plancher de la chambre sourde.

Selon une autre variante, le dispositif comprend deux et seulement deux assises disposées dans la chambre sourde, les assises étant accolées.

Selon encore une variante, le dispositif comprend deux écrans disposés face à face dans l'enceinte de part et d'autre des assises, les assises étant munies de dossiers et étant orientées de sorte que deux utilisateurs disposés dans les assises visionnent un écran distinct et puissent s'observer.

Selon encore une autre variante, le dispositif comprend une unique assise disposée dans la chambre sourde.

Selon une variante, la chambre sourde ne contient pas de source sonore.

Selon encore une variante, le l'écran est distant de parois latérales et d'un plancher de la chambre sourde.

Selon une autre variante, le dispositif comprend une source de flux vidéo appliquant un flux vidéo sur l'écran.

Selon encore une autre variante, la chambre sourde comprend six parois formant un parallélépipède rectangle, les parois étant mutuellement démontables.

Selon une variante, le dispositif comprend une toile recouvrant des parois latérales de la chambre sourde.

Selon encore une variante, le dispositif comprend une porte d'accès à la chambre sourde, et une borne de vérification de droit d'accès permettant de vérifier un droit d'accès et de commander sélectivement le déverrouillage de la porte d'accès. La porte d'accès peut être de différentes natures, notamment une porte à pivot, avec ou sans poignée, une porte coulissante, lesdites portes pouvant être manuelles, électriques ou électroniques.

D'autres caractéristiques et avantages de l'invention ressortiront clairement de la description qui en est faite ci-après, à titre indicatif et nullement limitatif, en référence aux dessins annexés, dans lesquels :
- la figure 1 illustre une chambre sourde selon un premier mode de réalisation de l'invention en coupe et en vue de dessus ;
- la figure 2 illustre une chambre sourde selon un deuxième mode de réalisation de l'invention en perspective.

Dans le cadre de la présente demande, une chambre sourde désigne une chambre anéchoïque ou semi-anéchoïque, c'est-à-dire présentant un degré d'isolation acoustique particulièrement élevé. Une chambre anéchoïque est une salle d'expérimentation dont les murs et le plafond sont totalement absorbants aux ondes sonores ou électromagnétiques, donc ne provoquent aucun écho venant perturber les mesures. Des propriétés acoustiques des chambres anéchoïques sont définies dans la norme ISO 3745, notamment les niveaux de bruits de fond et les lois de décroissance à 10 dB, de préférence à 6 dB. Cette norme définit notamment le niveau d'atténuation dans le spectre de perception sonore humain.

Ainsi, selon la norme ISO 3745, une chambre anéchoïque est de préférence une chambre qui, pendant l'essai de réception acoustique pour le niveau de pression sonore, la diminution de celui-ci fait l'objet de mesures techniques dans un champ libre sur une surface à réflexion acoustique, et pour chaque fréquence considérée, on s'attend à une diminution de 6dB par intervalle double.

Une chambre sourde selon l'invention est de préférence une chambre présentant un tel degré de diminution de pression sonore, tel que mesuré dans les conditions ci-dessus.

Pour le test, le haut-parleur est utilisé comme source sonore qui présente des caractéristiques de diffusion du son sphériques.

On préfère généralement que le centre acoustique soit inférieur à 0,1*λ au-dessus du sol de réflexion acoustique ou que la source sonore utilisée pour le test soit encastrée dans le sol.

Pour ce test, les haut-parleurs eux-mêmes ont une taille pouvant aller jusqu'à 35 cm. Les mesures sont effectuées dans une gamme de fréquence de 80 Hz à 16 kHz par tierce.

En option, des mesures peuvent être effectuées dans une gamme de fréquence plus étendue, à partir de 25 Hz.

Le son à la tierce est utilisé pour l'activation de la chambre.

La diminution du niveau de pression sonore avec la distance est de préférence enregistrée sur au moins 5 trajectoires émanant radialement de la source sonore.

Les différences entre les valeurs mesurées et les valeurs calculées avec la loi de l'inverse du carré sont déterminées.

La chambre à champ libre remplit les exigences de la classe de précision de la norme DIN EN ISO 3745, lorsque les différences ne dépassent pas la valeur limite minimale.

De ces différences, les positions de mesures admissibles peuvent être dérivées dans l'angle de mesure autour de la source, positions dans lesquelles règnent les conditions de champ libre requises. L'angle de mesure est limité par une distance au mur supérieure ou égale à λ/4 selon les fréquences.

Afin d'assurer le niveau d'isolation souhaité, les parois de la chambre sourde sont typiquement composées de dièdres (espèce de petites pyramides), généralement en mousses polymères ou fibres de verre, dont la particularité est d'absorber les ondes sonores. La qualité de l'absorption est fonction de la taille de ces dièdres et de la qualité du matériau exploité.

Selon une autre variante, les parois peuvent être réalisées par la technologie BCA ("Broadland Compact Absorber : the acoustic requirements of semi-anechoic and anechoic rooms up to frequencies of below 50 Hz"). La technologie BCA repose sur l'utilisation de modules de panneaux sandwich, qui sont assemblés à partir de blocs poreux quadrangulaires faits de matériau(x) absorbants et contenant une plaque de résonance interne. L'épaisseur de ces modules est généralement de 250 mm environ.

La chambre sourde peut être de forme variée, par exemple carrée, rectangulaire, ovale, ronde, etc. Ses dimensions peuvent être adaptées en fonction de l'usage. Le dispositif de l'invention peut être destiné à un usage public ou privé, et positionné en intérieur ou en extérieur.

L'invention propose ainsi un dispositif de restitution d'image, comprenant une chambre sourde, un écran d'affichage disposé dans la chambre sourde, une assise disposée dans la chambre sourde de sorte qu'un utilisateur puisse visionner l'écran et, de manière facultative, une tenue vestimentaire pour une isolation et/ou perception similaire des participants. Comme indiqué précédemment, un tel dispositif provoque chez chaque utilisateur non seulement une détente importante, mais également une perception visuelle totalement pionnière, unique et déclinable à l'infini suivant chaque être humain.

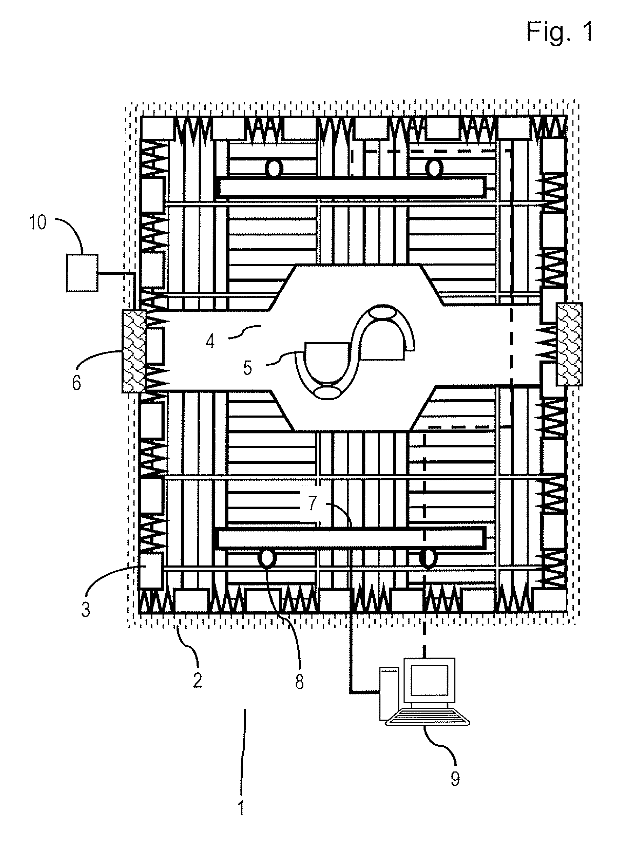

La figure 1 illustre plus précisément un exemple de mode de réalisation de l'invention. Le dispositif de restitution d'images 1 illustré comprend une chambre sourde, délimitée par des parois 2, recouvertes d'éléments d'absorption sonore 3. Les parois forment les murs, le sol et le plafond de la chambre sourde. Dans cet exemple, la chambre sourde a une forme de parallélépipède rectangle formé de six parois mutuellement démontables. La chambre anéchoïque pouvant être de forme ovale, ronde ou carré. Ainsi, le dispositif 1 peut être déplacé à souhait sur différents lieux, par exemple des foires ou des expositions. On peut également avantageusement dimensionner la chambre sourde pour que celle-ci soit grutable alors son matériau pour les parois extérieures pourrait être métallique pour faciliter son nomadisme.

La structure des éléments d'absorption 3 est connue en soi. Dans l'exemple illustré, les éléments d'absorption 3 sont formés de dièdres disposés selon deux orientations perpendiculaires sur chaque paroi. Afin d'éviter que des utilisateurs ne dégradent les éléments d'absorption 3, on peut envisager de recouvrir ceux-ci d'un voile. Lorsque les éléments d'absorption 3 sont recouverts d'un voile, la définition du décor intérieur de la chambre sourde est beaucoup plus facile à réaliser. Les éléments d'absorption 3 ou le voile le cas échéant, peuvent être d'une couleur bleue foncée, afin de relaxer les utilisateurs et les placer dans une ambiance les poussant à la rêverie. Les éléments d'absorption 3 et le voile peuvent être réalisés en matériaux ignifugés.

Une banquette 5 présente deux assises pour des utilisateurs. La banquette forme également un dossier pour chaque assise. Les assises et les dossiers sont orientés de sorte que des utilisateurs présents sur les assises aient le regard orientés dans des orientations opposées, tout en pouvant s'observer ou des orientations similaires. Les assises et les dossiers sont avantageusement configurés pour limiter les efforts musculaires de l'utilisateur et faciliter son relâchement physique. La banquette 5 est disposée sur une plateforme 4 suspendue au dessus du plancher de la chambre sourde. Ainsi, les utilisateurs peuvent avoir une impression d'être suspendus dans un volume. On peut également envisager que la banquette 5 soit surélevée par rapport à la plateforme 4. La plateforme 4 ou la banquette 5 peuvent être montés sur des ressorts par rapport au plancher de la chambre sourde. On pourrait également envisager que la plateforme recouvre l'intégralité du plancher de la chambre sourde, de sorte que l'utilisateur dispose alors d'une grande liberté de déplacement. La plateforme 4 peut être recouverte de moquette pour créer une ambiance relaxante pour l'utilisateur.

Afin de limiter les bruits parasites, la ou les assises sont de préférence revêtues d'un matériau tel que du feutre, afin de générer un minimum de bruits lors de mouvements de l'utilisateur. La ou les assises peuvent également être fabriquées en mousse viscoélastique à mémoire de forme. La ou les assises peuvent être montées pivotantes par rapport au plancher de la chambre sourde.

Deux portes 6 permettent de pénétrer dans la chambre sourde depuis l'extérieur. Les portes 6 sont disposées de part et d'autre de la banquette 5. La présence de portes 6 de part et d'autre permet aux utilisateurs d'évacuer aisément la chambre sourde. Le dispositif 1 comprend une borne de vérification de droit d'accès 10. La borne 10 permet de vérifier si un utilisateur a droit d'accéder à la chambre sourde, puis de commander sélectivement le déverrouillage d'une des portes 6 en fonction de la vérification (l'ouverture des portes électroniques se commandant depuis le fauteuil en activant un bouton sur la borne et cette ouverture électronique se jumelle à une ouverture manuelle des portes). La borne 10 peut également servir à choisir un programme de diffusion parmi les catalogues proposés : cinéma, art plastique, photographie, littérature, etc....). La borne 10 peut également permettre à l'utilisateur de définir la durée de chaque séance d'affichage d'un flux vidéo dans la chambre sourde. Cette durée pourra être prédéfinie et être comprise entre 5 et 20 minutes par exemple. Dans un mode de mise en oeuvre, il est prévu que le spectacle audiovisuel s'interrompe automatiquement après 7 minutes, permettant au spectateur de bénéficier d'une pause recommandée dans le sas de conditionnement, ou mettant terme à la séance.

Avantageusement, la borne 10 est disposée dans un vestibule donnant accès à une des portes 6. Afin de créer une ambiance relaxante pour l'utilisateur, on peut envisager que de la musique soit diffusée dans le vestibule. De plus, la diffusion de musique dans le vestibule permet de créer un contraste sonore lorsque l'utilisateur pénètre dans la chambre sourde. Ce sas de conditionnement ou sas de relaxation ou de décompression psychologique peut volontiers aussi être envisagé sans musique, comme une étape qui conduit au silence profond totalement étranger aux spectateurs imminents de ce dispositif.

La chambre sourde illustrée présente deux écrans 7 disposés face à face. Les écrans 7 sont disposés de part et d'autre de la banquette 5. Avantageusement, les écrans 7 sont distants des parois latérales de la chambre sourde. Outre l'aspect esthétique, une telle disposition des écrans peut améliorer les propriétés acoustiques de la chambre sourde. On peut notamment envisager que les écrans 7 soient suspendus au plafond de la chambre sourde, à distance du plancher de la chambre sourde (et donc entouré d'air). Dans l'exemple illustré, les écrans 7 sont fixés au plafond de la chambre sourde par l'intermédiaire de barres 8. On peut également envisager que les écrans soient encastrés dans les parois latérales de la chambre sourde. La taille de ces écrans occupe avantageusement les parois latérales pour renforcer l'immersion.

Bien que les écrans 7 illustrés soient sensiblement plats, on peut également envisager des écrans en forme de portions de cylindres. On peut également envisager un écran couvrant toute la périphérie de la chambre sourde, combiné à une assise pivotante, permettant à l'utilisateur de choisir la partie de l'écran qu'il souhaite visionner. Le flux vidéo affiché peut également être prévu pour obtenir des effets de stéréoscopie et de stéréocinétique en jouant sur les phénomènes de mouvements, couleurs, transparence avec plusieurs écrans se coulissant, des effets de trompe l'oeil, de stéréoscopes à miroir (Wheatstone) ou à prisme (Brewster), des anaglyphes, les techniques d'observation des autostéréogrammes et illusion du papier peints (vergence binoculaire sur des plans situés en dehors de celui du stimulus et convergence en deçà du plan) pour obtenir de phénomènes de tridimensionnalité, utilisation de l'illusion de Pulfrich, de stimulations synesthésiques, etc.).

L'invention est spécialement adaptée au cinéma "futuroscopique", à savoir, en relief, dynamique ou les deux.

Pour jouer sur les effets tridimensionnels, il est préférable que la distance écran-spectateur soit en dessous de 1m50 (distance maximum de 6 mètres).

L'homme du métier pourra déterminer la technologie d'affichage de l'écran parmi les nombreuses technologies actuellement disponibles : utilisation d'un projecteur, vidéo projecteur, écran SED, écran OLED, écran à cristaux liquides, écran à plasma, écran à rétroprojection, écran laser, diffusion cinéma numérique, etc.

Pour un effet optimal, il est important que l'écran soit très lumineux et que le reste ne le soit pas. Dans ce but, dans une variante de l'invention, les murs, sols, et assises sont avantageusement recouverts de matériau(x) de surface(s) appelé(s) corps noirs. Ces matériaux absorbent la lumière provenant de l'écran.

Le dispositif 1 comprend une source de flux vidéo 9. La source 9 est connectée aux écrans 7 pour leur appliquer des flux vidéo. Le flux vidéo issu de la source 9 peut être modifié en fonction de commandes transmises depuis une assise ou en fonction d'une interface de commande à disposition d'un utilisateur placé à l'extérieur de la chambre sourde. Différents flux vidéo peuvent être projetés afin de créer une ambiance relaxante pour l'utilisateur. On peut également envisager de diffuser un flux vidéo à environ 10 images par secondes (lissage anisotrope), ce qui crée un effet relaxant pour l'utilisateur. Le flux vidéo peut être sélectionné par l'utilisateur. Le flux vidéo peut également être affiché sans que l'utilisateur en ait une connaissance préalable, par exemple dans le cas d'un flux vidéo généré aléatoirement. La perception d'images dans un silence profond ainsi que le choix des flux vidéo pourront notamment rapprocher la vision de l'utilisateur de celle générée par le cerveau en phase d'endormissement, caractérisée par une sensation de flottement. Le flux vidéo pourra notamment viser à créer une vision holographique, mouvante et onirique. On peut encore envisager que la source 9 commande l'affichage de photos ou de textes, par exemple sous forme de diaporamas.

Le cerveau devant être stimulé après 2 minutes pour que l'effet recherché dure, les documents présentés sont avantageusement conçus spécialement pour l'invention, et réalisés sous le contrôle des 2 laboratoires de recherche scientifique en Neurophysiologie et en Psychologie de la Perception.

Dans l'hypothèse où la chambre sourde est munie de sources sonores, l'utilisateur pourra disposer d'une commande permettant de faire varier le volume pour jouer sur la relativité du silence. On pourra notamment envisager que les sources sonores permettent de restituer un niveau sonore maximum de 25 dB dans la chambre sourde, afin de garantir une relativité du silence.

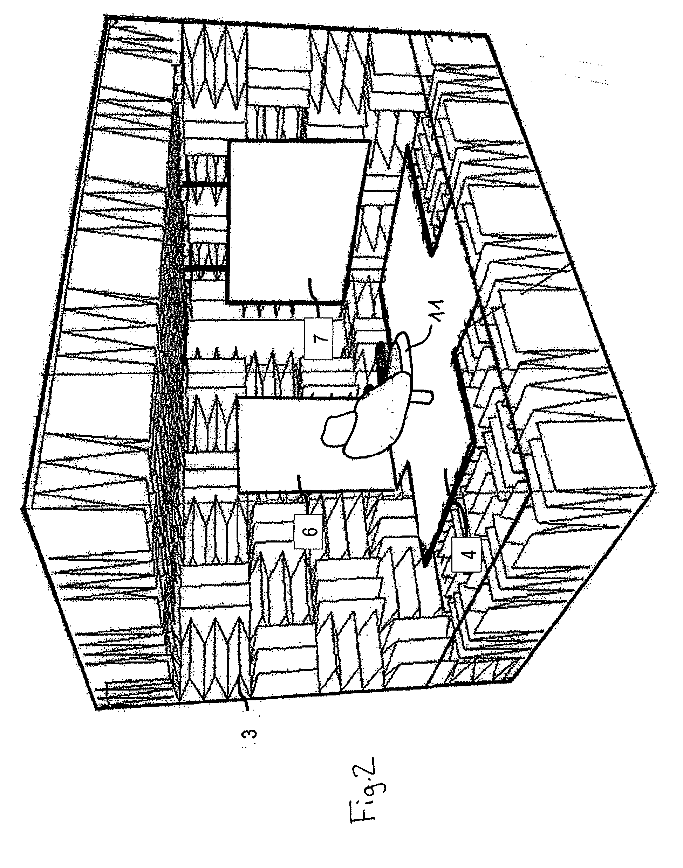

Dans l'exemple illustré à la figure 2, la chambre sourde n'inclut qu'un seul fauteuil 11, monté pivotant par rapport à la plateforme 4. Le fauteuil est monté de sorte que l'utilisateur puisse visionner l'écran 7.

Afin de présenter un cadre reposant pour les utilisateurs, la chambre sonore ne présente avantageusement aucune source sonore. L'utilisateur est alors en présence d'un silence profond qui accroît sa perceptivité, notamment du fait que ce silence n'existe nulle part ailleurs que dans cette chambre anéchoique. L'influence sur le cerveau des flux vidéo affichés sur l'écran est alors accrue. L'utilisateur perçoit en outre de façon exacerbée des sons qu'il est peu habitué à remarquer : respiration, déglutition, battements cardiaques, etc. L'activité cérébrale de l'utilisateur est alors différente, son état de conscience étant modifié. On peut également envisager d'intégrer un micro dans la chambre sourde et de l'associer à une source sonore de génération de sons en opposition de phase avec les sons perçus par le micro. Un tel dispositif permet de maintenir de façon dynamique le silence à l'intérieur de la chambre sourde.

La chambre sourde est avantageusement prévue pour ne présenter que deux assises au maximum, afin de permettre aux utilisateurs présents dans la chambre sourde de ne pas être dérangés par la présence ou les bruits générés par d'autres utilisateurs. Les dimensions intérieures d'une chambre sourde seront avantageusement une hauteur d'au moins 2300 mm, une longueur supérieure à 3000 mm, ou une largeur supérieure à 2500 mm. On peut notamment envisager des dimensions intérieures avec une hauteur de 2400 mm, une longueur de 4560 mm, une largeur de 3360 mm. Bien entendu, des dimensions plus petites peuvent être envisagées également, pour atteindre des particuliers ou des sites d'implantation plus petits.

Des éléments lumineux sont avantageusement disposés dans le plafond de la chambre sourde. Une lumière adéquate sera choisie pour favoriser un cadre apaisant pour l'utilisateur. Avantageusement, les sources lumineuses ne sont pas visibles directement depuis la ou les assises. La lumière issue des sources lumineuses peut alors être réfléchie ou diffusée sur le trajet de la source lumineuse, de sorte qu'un utilisateur présent dans la chambre sourde perd partiellement ses repères dans l'espace.

Une ventilation ou une climatisation peut avantageusement déboucher dans la chambre sourde. L'homme du métier saura déterminer une ventilation ou une climatisation adéquate pour limiter le volume sonore généré dans la chambre sourde et respecter les normes, paramètres et mesures de la chambre.

L'utilisation d'un dispositif de l'invention plonge l'utilisateur dans un environnement unique qui transforme la perception visuelle, produisant sur chacun une sensation d'immersion et de réalité virtuelle. Au delà de la sensation de détente provoquée, la perception d'images dans un dispositif de l'invention aborde des notions d'esthétiques et rapproche la vision de l'utilisateur de celle générée par le cerveau en phase d'endormissement caractérisée par une sensation de flottement, une vision holographique, mouvante et onirique. Le dispositif entraîne la perception d'images hypnagogiques ou corticales et un état de conscience modifié. Le spectateur va atteindre l'état de conscience n°1 (veille éveil) et l'état de conscience n°2 (endormissement) et bénéficier de la présence des ondes cérébrales BETA (13 HZ et plus) et ALPHA (8 à 12 HZ) en dissociant les 3 éléctros : encéphalogramme, occulogramme (yeux roulement de droite à gauche), éléctromiogramme (tonus musculaire). Le dispositif de l'invention est adapté pour une installation en intérieur ou en extérieur.

## Revendications

1. Dispositif de restitution d'images (1), **caractérisé en ce qu'**il comprend :
- une chambre sourde anéchoïque ou semi-anéchoïque ;
- un écran d'affichage (7) disposé dans la chambre sourde ; et
- une assise (5) disposée dans la chambre sourde de sorte qu'un utilisateur puisse visionner l'écran.

2. Dispositif selon la revendication 1, comprenant une plate-forme (4) sur laquelle est fixée l'assise, la plate-forme étant suspendue au dessus d'un plancher de la chambre sourde.

3. Dispositif selon la revendication 1 ou 2, comprenant deux et seulement deux assises disposées dans la chambre sourde, les assises étant accolées.

4. Dispositif selon la revendication 3, comprenant deux écrans (7) disposés face à face dans l'enceinte de part et d'autre des assises, les assises étant munies de dossiers et étant orientées de sorte que deux utilisateurs disposés dans les assises visionnent un écran distinct et puissent s'observer.

5. Dispositif selon la revendication 1 ou 2, comprenant une unique assise disposée dans la chambre sourde.

6. Dispositif selon l'une quelconque des revendications précédentes, dans laquelle la chambre sourde ne contient pas de source sonore.

7. Dispositif selon l'une quelconque des revendications précédentes, dans laquelle l'écran (7) est distant de parois latérales et d'un plancher de la chambre sourde.

8. Dispositif selon l'une quelconque des revendications précédentes, comprenant une source (9) de flux vidéo appliquant un flux vidéo sur l'écran.

9. Dispositif selon l'une quelconque des revendications précédentes, dans lequel la chambre sourde comprend six parois formant un parallélépipède rectangle, les parois étant mutuellement démontables.

10. Dispositif selon l'une quelconque des revendications précédentes, comprenant une toile recouvrant des parois latérales de la chambre sourde.

11. Dispositif selon l'une quelconque des revendications précédentes, comprenant une porte d'accès (6) à la chambre sourde, et une borne (10) de vérification de droit d'accès permettant de vérifier un droit d'accès et de commander sélectivement le déverrouillage de la porte d'accès.

12. Dispositif selon l'une quelconque des revendications précédentes, comprenant en outre une tenue vestimentaire spécifique permettant de diminuer les sons émis par l'utilisateur et/ou que tous les individus entrant dans le dispositif soient vêtus pareillement.

13. Dispositif selon l'une quelconque des revendications précédentes, **caractérisé en ce que** les murs, sols, et assises de la chambre sourde sont avantageusement recouverts de corps noirs.
